# EUROPEAN PATENT APPLICATION

(11) **EP 0 606 553 A2**
(43) Date of publication of application: **20.07.1994**
(21) Application number: 93117958.4
(22) Date of filing: 05.11.1993
(51) Int. Cl.: C10M 105/34, C10M 105/38, C10M 105/42, C10M 177/00, C09K 7/00, C10N 70/00

(54) **An ester base oil for lubricant compounds and process of making an ester base oil from an organic reaction by-product**

(30) Priority: 11.01.1993 US 3089
(71) Applicant: CONOCO INC., Ponca City, Oklahoma 74602-1267 (US)
(72) Inventor: Durr, Albert M., Jr., Ponca City, Oklahoma 74601 (US); Huycke, John, Houston, Texas 77095-3654 (US); Jackson, Harold L., Hockessin, Delaware 19707 (US); Hardy, Bryant J., Ponca City, Oklahoma 74604 (US); Smith, Kenneth W., Ponca City, Oklahoma 74604 (US)
(74) Representative: Woodman, Derek

(57) **Abstract**

This application relates to a process for reacting an organic reaction by-product to form an ester lubricant base oil, and the composition of the complex ester obtained by the process. The by-product material is the non-volatile residue of the oxidation reaction of cyclohexane to make intermediates for the synthesis of adipic acid.

## Description

### Background of the Invention

This application relates to the composition of an ester lubricant base oil and a process for making the ester lubricant base oil from a by-product of an organic reaction. The esters of the invention are synthesized by reacting one or more alcohols and/or polyols with a non-volatile residue from the manufacture of intermediates of adipic acid under conditions suitable for forming esters. The reaction product is a complex ester, (i.e., a mixture of esters,) which may be used directly as a lubricant, or may optionally be further compounded and treated to form greases or other lubricants.

The ester lubricant base oil of the invention may be used by itself, or with appropriate additives, as a lubricant for gears, motors, compressors, turbines, internal combustion engines, or as a drilling fluid or machine oil. The ester base oil may also be compounded to form grease which may be used as bearing, gear, chain, or rail lubricants, for example.

Esters are well known as lubricants and are suitable for a wide variety of applications. For example, World Patent Application No. WO 14402/'90 A1 describes the use of selected ester oils in invert emulsion drilling fluids having improved environmental acceptability. Other uses for ester based oils are presented in the book Synthetic Lubricants and High-Performance Functional Fluids, (Marcel Dekker, New York, 1993). Ester base oils form a substantial segment of the current lubricant market. These esters, while providing excellent lubricating properties, are comparatively high-priced on account of the price of the starting materials from which the esters are synthesized.

The inventors found that the by-product from the manufacture of intermediates of adipic acid is rich in carboxylic acids desirable for making an ester lubricant base oil. With little or no pre-treatment, the by-product can be reacted with alcohols and/or polyols under ester forming conditions to produce esters having lubricant properties. The ester base oil made according to the invention has a great advantage in economy over other ester base oils in that it is obtained from an abundant reaction by-product which would otherwise be used as fuel.

### Detailed Description of the Invention

One way in which esters may be formed is by the reaction of a carboxylic acid and alcohols and/or polyols through the process known as esterification. An ester is formed when the carboxyl hydrogen of the acid is replaced with the alkoxy moiety (R'O) from the alcohol or polyol. Water is a by-product of the esterification reaction. The esterification reaction is generically illustrated by general formula (i) presented below:

(i) RCOOH + R'OH ⇄ RCOOR' + H₂O

This is the primary reaction by which the esters of the invention are formed.

The carboxylic acids used in the reaction of this invention are the principal components of NVR. NVR is obtained as a by-product of the air oxidation of cyclohexane to form cyclohexanol and cyclohexanone, intermediates in the synthesis of adipic acid. The by-product is collected as the non-volatile residue of the bottoms of the steam distillation of the cyclohexane and cyclohexanol product. The name NVR is obtained from the term "non-volatile residue". Sometimes the by-product is also referred to as "adipic NVR". Chemical Abstracts has assigned CAS number 6841-76-7 to NVR.

NVR is comprised chiefly of C₄, C₅, and C₆ monobasic, dibasic, and hydroxy aliphatic carboxylic acids and alcohols. TABLE 1 (below) provides a typical composition of NVR and the approximate weight percentage of the major organic components of the material. In practice, the composition of NVR is variable, and a proportion of the components may be present as ester oligomers.

**TABLE 1**

| **Component** | **Wt.%** |
|---|---|
| 6-hydroxycaproic acid | 26.8 |
| valeric acid | 13.8 |
| adipic acid | 11.9 |
| 5-hydroxyvaleric acid | 11.0 |
| 4-hydroxybutyric acid | 6.4 |
| caproic acid | 4.4 |
| 2-hydroxycyclohexanone | 4.4 |
| n-pentanol | 4.0 |
| butyric acid | 3.1 |
| caproaldehyde | 2.6 |
| glutaric acid | 2.0 |
| dicylohexyl peroxide | 2.0 |
| dicylohexyl ether | 2.0 |
| cyclopentanol | 1.1 |
| cyclohexyl hydroperoxide | 0.9 |
| n-butanol | 0.7 |
| succinic acid | 0.5 |
| cyclohexanone | 0.5 |
| formic acid | 0.3 |
| cyclohexenone | 0.2 |
| cyclopentanone | 0.2 |
| valeraldehyde | 0.2 |
| acetic acid | 0.2 |
| cyclohexanol | 0.1 |
| other substances (excluding water) | 0.7 |

Note that the C₄-C₆ carboxylic and hydroxycarboxylic acids (6-hydroxycaproic acid, valeric acid, adipic acid, 5-hydroxyvaleric acid, and 4-hydroxybutyric acid) account for between 75-80 wt % of all the carboxylic acids in NVR. Also note that the composition presented in TABLE 1 is the typical "dry" or anhydrous composition of NVR. (Because of the steam distillation process by which the NVR is separated from the adipic intermediates, a substantial amount of water is present in the "wet" NVR. The amount of water in the "wet" NVR ranges from about 15-25 vol %).

The esterification reaction may be carried out with the NVR in its "wet" form. However, the NVR may optionally be dewatered prior to the esterification reaction. Satisfactory dewatering may be accomplished by distilling the wet NVR at a temperature in the range of about 100-150°C in order to drive the water from the NVR. As some steam volatile organics will likely be distilled-off with the water, the distillation vapor may be condensed and the organics separated and returned to the NVR.

It will be appreciated that the alcohols and polyols to be reacted with the NVR may be used individually or as mixtures. That is, a single type of alcohol or a single type of polyol may be reacted with the NVR, or two or more alcohols and/or polyols may be freely mixed and reacted with the NVR. The alcohols and polyols suitable for reaction with NVR include mono-alcohols illustrated by general formula (ii), neopentylpolyols illustrated by general formulas (iii) and (iv), linear diols illustrated by general formula (v), polyglycols illustrated by general illustrated by general formula (vi), and polyglycerines illustrated by general formula (vii) as presented below:

(ii) mono-alcohols R¹OH

wherein R¹ is an alkyl group comprising from 1-25 carbons which can be straight, branched or cyclic in structure.

Such mono-alcohols include one or more alcohols selected from the group consisting of methanol, ethanol, propanol, butanol, pentanol, hexanol, heptanol, octanol, nonanol, decanol, undecanol, and dodecanol, wherein the alkyl portion of the alcohol may be straight, branched or cyclic. Some specific examples of alcohols suitable for reaction with NVR according to the present invention include, but are not limited to, 1-decanol, 1-dodecanol, 2-methyl-1-propanol, 2-ethyl-1-hexanol, 1-butanol, the C₆ oxo-alcohols, and the C₈ oxo-alcohols. 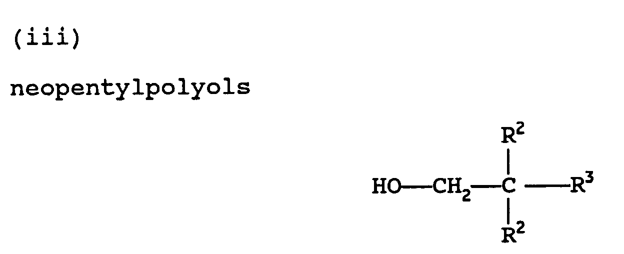
wherein R² represents one or more members selected from the group consisting of hydrogen, a C₁ - C₆ alkyl which is straight chained or branched, and CH₂OH, and R³ represents a C₁ - C₃ alkyl group (methyl, ethyl, and propyl) which is straight or branched (such as n-propyl or i-propyl), or CH₂OH. Some specific examples of these alcohols are 2,2-dimethyl 1,3-propanediol, 1,1,1-tris (hydroxymethyl) ethane, and 2-ethyl-2-(hydroxymethyl)-1,3propane diol. 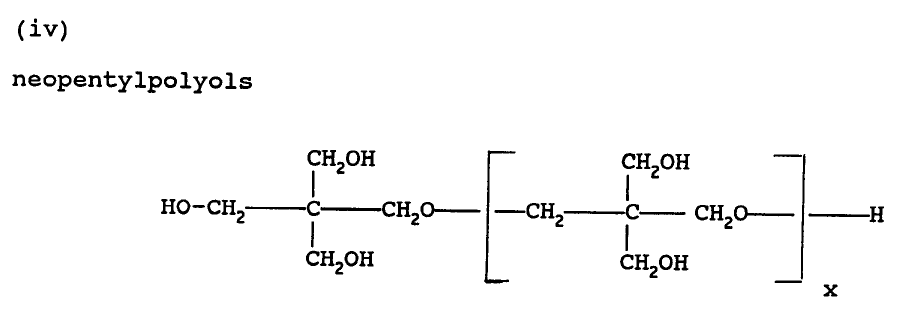
wherein **x** is an integer of from 0 to 4.

(v) linear diols HO-[R⁴O]_{z}H

wherein R⁴ represents a C₁-C₁₀ alkyl group which is linear or branched; and **z** is an integer of from 1 - 100. It is preferred that **z** is an integer of from 1 - 20. It is also preferred that R⁴ represent a C₁-C₆ alkyl group which is linear or branched. Specific linear diols described by general formula (v) include ethyleneglycol, propyleneglycol, butyleneglycol, pentyleneglycol, hexyleneglycol, heptaleneglycol, octaleneglycol, nonaleneglycol, decaleneglycol, and the "poly" variations of each when the [R⁴O] moiety repeats. 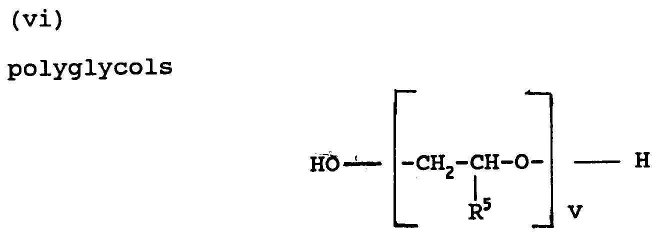
wherein R⁵ represents H, or a C₁-C₆ alkyl group which may be straight or branched, and **v** represents an integer of from 1 - 50, with 1 - 20 being a preferred range. 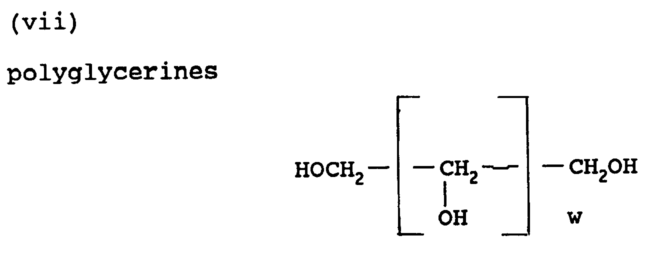
wherein **w** represents an integer of from 1 - 20.

The reaction between NVR and the selected alcohols and/or polyols is carried out by heating the reactants under conditions suitable to promote the esterification reaction. Such conditions preferably include heating the reactants in the presence of a catalyst for the esterification reaction.

Suitable catalysts include strong acid catalysts selected from the group consisting of sulfonic acid, phosphoric acid, p-toluenesulfonic acid, xylenesulfonic acid, methane sulfonic acid, and sulfuric acid. The preferred strong-acid catalyst of the inventors is p-toluenesulfonic acid. It will be appreciated by those skilled in the art that these strong acids are sometimes referred to as soluble acids due to their ability to dissolve in the esterification reaction mixture.

Suitable esterification catalysts also include solid acid (i.e., water insoluble) catalysts. Such solid acid catalysts are exemplified by sulfonated polystyrene macro-reticular acidic ion exchange resins. Such a solid acid catalyst is commercially available from Rohm & Haas Co. of Philadelphia, Pennsylvania, under the trademark AMBERLYST 15. A solid acid catalyst is the preferred catalyst for use in the present invention.

Suitable esterification catalysts also include non-acid catalysts. Additionally, non-acid catalysts also have utility in catalyzing the trans-esterification reactions necessary to build esters of the appropriate molecular weight and size when larger esters are required. Non-acid catalysts may include one or more of the materials selected from the group consisting of alkyl titanates, zinc chloride, tin chloride, sodium bisulphate, and potassium bisulfate.

The amount of soluble acid catalyst used in the invention ranges from 0.01 to 2.0 wt% of the total weight of the reactants. The most preferred amount of soluble acid catalyst is approximately 0.1 wt% of the total weight of the reactants. The amount of non-acid catalyst used to promote the reaction ranges from 0.005 to 0.1 wt.% of the total weight of the reactants. No specific weight range of solid acid catalyst has been determined.

When preparing the esters of the invention using batch processing techniques and a suitable soluble acid catalyst, the reaction conditions require the operator to maintain the reactants at a temperature sufficient to promote the esterification reaction. Although it is well known in the art that the esterification reaction can occur over a very broad temperature range, such as from 0-300°C, it is believed that the esterification reaction is best practiced in the range of from 180-250°C. Maintaining the reaction in this temperature range provides a reasonable to good rate of reaction and better control over the viscosity of the finished ester product. For example, reactions carried out below 180°C may tend to be too slow for commercial use and esterification reactions with NVR and pentaerythritol carried out at temperatures in excess of approximately 250°C produced rubbery, polyester solids unsuitable for use as lubricants.

Depending upon reaction conditions, the reactant alcohols and/or polyols selected, and the degree of transesterification which is permitted to take place, the esters prepared by this reaction may range from low viscosity esters having a low molecular weight and high volatility to very viscous esters which have the consistency of rubbery solids. By manipulating the variables described above the practitioner can tailor the ester viscosity to suit his particular needs. The esters which are believed to have the highest value as lubricants are those esters which have a viscosity in the range of from approximately 3 - 200,000 cSt at 25°C.

The majority of ester products of the reaction are believed to be represented by general formula (viii) as follows: 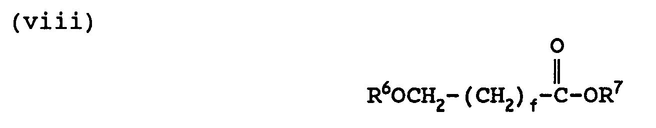
wherein R⁶ represents one or more of the moieties selected from the group consisting of:

-H ;

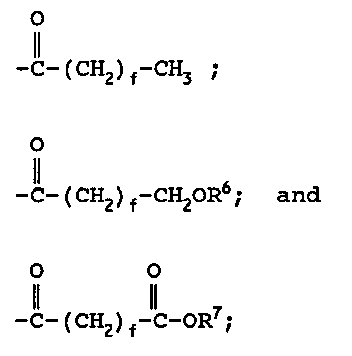

and wherein R⁷ represents one or more of the moieties selected from the group consisting of:

-CH₂-(CH₂)_{f}-CH₃;

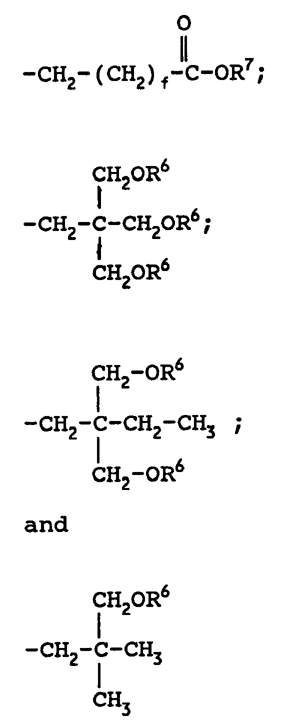

and **f** represents integers of from 2 through 4.

It will be appreciated by those skilled in the art that esters can be formed by molecules within the NVR reacting among themselves. This occurs because NVR contains materials having carboxylic acid groups and materials having hydroxyl groups. Because the carboxylic acid groups in the NVR are in a stoichiometric excess of the hydroxyl groups, additional alcohol must be added to the NVR to carry out a more complete conversion of the carboxylic acids to esters.

The esterification reaction of the invention may be driven to virtual completion by continuously removing the water produced as a by-product of the reaction. Removing by-product water pushes the reaction equilibrium in the direction of the ester product, thereby improving reaction yields. This may be accomplished by means of a distillation step incorporated into the esterification process itself which permits the water vapor to be drawn-off while the NVR is undergoing the esterification reaction. Incorporating the distillation step in the reaction process is most feasible when very high boiling point alcohols are being reacted with the NVR. Examples of these high boiling point alcohols include neo-pentyl polyols, 2-ethyl-1-hexanol, and n-decanol.

The process of the invention permits a great deal of flexibility in selecting the viscosity of the esters synthesized. Higher molecular weight esters may be produced by reacting alcohols of low volatility with the NVR and/or by displacing an alcohol of a higher volatility from the original ester molecule during the reaction process. The displacement process is known as transesterification.

Ester lubricants with viscosities greater than 35 cSt (at 100°C) can be prepared using a batch method wherein all of the components of the reaction are added simultaneously to a single reaction vessel. This method is used primarily when the alcohol is relatively non-volatile, such as when using neopentyl polyols (e.g., pentaerythritol, trimethylolpropane, or neopentyl glycol). The composition of the mixture is adjusted so that the amount of added alcohol is sufficient to convert all of the free acid components of the NVR. A large molar excess of the added alcohol is avoided so as to not have significant quantities of unreacted alcohol remaining in the final product. The reactants (NVR, alcohol, and catalyst) are mixed continuously in the reactor while the mixture is heated to boiling (approximately 100°C).

Vapors produced by boiling the reactants are drawn off the reaction vessel and condensed. The content of the overhead vapors is primarily water and C₄-C₆ monobasic carboxylic acids which may themselves be reacted with alcohols and/or polyols described herein by general formulas (ii), (iii), (iv), (v), (vi), and (vii) to form esters. The esters made from these NVR overhead C₄-C₆ monobasic acids are of particular use as base oils for drilling muds, especially when the NVR overhead monobasic acids are reacted with 2-ethyl-1-hexanol and/or neopentyl glycol. Similar monobasic acids of C₄-C₆ may be obtained from NVR by the alternative process of adding soluble catalyst to NVR and adjusting the water content to approximately 25 wt%. This mixture is then boiled to distill off the water and steam volatiles, leaving a separable organic layer which is rich in monobasic acids which may be recovered by simple mechanical means, such as decanting. The monobasic acids derived in this manner may likewise be reacted with any of the alcohols and/or polyols represented herein by general formulas (ii), (iii), (iv), (v), (vi), and (vii). However, for the purposes of making esters suitable for use in drilling muds, the monobasic acids may be reacted with 2-ethyl-1-hexanol and/or neopentyl glycol.

Heat is continuously applied until the reaction mixture reaches a temperature of from 180-210°C. A vacuum of from 5-50mm Hg is applied to the reaction vessel to strip excess volatile acids and alcohols from the reaction mixture. The viscosity of the mixture is continuously monitored during this period until the desired target viscosity is reached through the reaction mixture undergoing esterification and transesterification reactions. When the target viscosity is reached the reaction mixture is rapidly cooled to quench the reaction. The product of the reaction is then treated with caustic, lime, and filtered, or alternatively, treated with an alkyl amine to remove and/or neutralize the catalyst and any residual acid components.

Ester lubricant base oils with viscosities between 5 and 50 cSt (at 100°C) prepared from the reaction of C₄-C₁₄ primary alkanols, polyols, or mixtures thereof having moderate volatility, such as n-butanol, 2-methyl 1-propanol, C₈ oxo-alcohols, 2-ethyl 1-hexanol, neopentyl glycol, trimethylol propane, pentaerythritol, and ethylene glycol.

In the manufacture of esters according to the invention, the excess alcohol can be easily removed from the reaction mixture via atmospheric or vacuum distillation. In the batch process, the alcohol, NVR, and catalyst are charged to the reaction vessel in a single step. The alcohol concentration is adjusted to be in significant excess over the amount required for the complete conversion of the acid functional groups in the NVR. The reaction mixture is heated with continuous agitation to boiling at approximately 100°C. The volatile components released by the boiling reaction mixture (water and alcohol) are collected from the reaction vessel and condensed. The alcohol distillate obtained from the boiling reaction mixture is separated from the aqueous distillate and the alcohol distillate is returned to the reaction vessel. The reaction mixture is continuously heated in order to keep the mixture at its boiling point. The rate of production of the water of reaction or the acid number of the reaction mixture, can be monitored to determine when the initial esterification process is complete. Initial esterification is complete when the water of reactions ceases or when the acid number drops below 5 mg KOH/g.

On completion of the initial esterification a moderate vacuum of 5-50 mmHg is applied to the reaction vessel to remove excess volatile alcohols and esters formed in the reaction. Removal of these materials helps to shift the reaction equilibrium of the process in favor of higher molecular weight ester oligomers by promoting transesterification reactions. During this portion of the process the viscosity of the reaction mixture is closely monitored until the desired viscosity is achieved. Once the target viscosity is achieved, the vacuum is removed, the mixture cooled, and the catalyst and excess acids are neutralized and/or removed with caustic or lime treatment and filtering.

The reaction of the invention may also be carried out through the use of an azeotropic reagent such as toluene or xylene which assists in the removal of water from the reaction mixture. After adding the azeotropic reagent, the reaction mixture is boiled to drive off the reagent and associated water vapor. The vapors are collected and condensed and the azeotropic reagent separated from the water by atmospheric distillation and returned to the reaction mixture. Once the desired viscosity of the ester mixture is achieved, the mixture is vacuum stripped and neutralized.

Alcohols having high volatility, i.e. those alcohols having atmospheric pressure boiling points below 100°C, can also be used in the reaction of the invention. High volatility alcohols can be used to produce NVR based esters with a viscosity in the range of from 5-50 cSt (at 100°C), however, the batch processing techniques are different than those used for other alcohols on account of the volatility of the alcohol reactant.

To produce esters the NVR, alcohol, and catalyst are charged to the reaction vessel equipped to permit the introduction of additional alcohol during the reaction process. The vessel is heated to a temperature in the range of from 120°-140°C and additional alcohol is pumped into the vessel at a rate which does not cause the reaction mixture temperature to drop below 120°C. Vapors produced by the reaction mixture are withdrawn overhead and condensed and the reaction is carried out until the acid number of the mixture drops to less than 5 mg KOH/g. A moderate vacuum of 5-50 mmHg is then applied to the reaction mixture to remove excess alcohol and volatile esters.

On removal of the alcohol, the equilibrium of the reaction is shifted in the direction of higher molecular weight ester oligomers via transesterification reactions. Viscosity of the reaction mixture is continuously monitored until the desired viscosity is achieved. On reaching the target viscosity, the vacuum is removed, the mixture cooled, and the acids and catalysts removed or neutralized with caustic or lime and filtering.

The reaction of the invention may also be practiced using a continuous process method. One way in which the invention may be practiced is through the use of a continuous packed bed reactor process. In this instance, an insoluble resin acid catalyst (e.g., a cation exchange resin in the acid form) can be used very effectively. The continuous packed bed method is most useful when high volatility alcohols, such as methanol, are used. However, the packed bed reactor method may also be used with less volatile alcohol reactants.

To make esters using a continuous packed bed reactor process a mixture of alcohol and dewatered NVR is pumped through a packed bed of acid cation exchange resin, wherein alcohol is present in the mixture in a very large excess. For example, successful esterification reactions have been carried out using a packed bed reactor process where the reaction mixtures comprised up to 50% methanol and 50% dewatered NVR.

The bed volume of the reactor should be sufficient to allow the space velocity of the reaction mixture to reach 1 - 4 bed volumes per hour. The effluent of the reactor can be fed to a continuous atmospheric still and fractionating column where the excess alcohol, water of reaction, and volatile esters are removed from the reaction product. The excess alcohol can be recycled back to the esterification step.

Volatile esters recovered from the reaction mixture can be used as a byproduct chemical feedstock, or used as a solvent. The bottoms of the atmospheric still are fed to a vacuum still and fractionating column where the vacuum distillates having viscosities appropriate for synthetic base oils are collected. The materials produced are linear oligomeric esters made up of segments comprised of diacids and hydroxy acids, with the ends of the oligomers capped with ester functions.

The process known as reactive distillation can be used to prepare low viscosity oils from NVR and C₄-C₈ alkanols. Reactive distillation is carried out by using a segment of a distillation fractionating tower as a reaction zone which is packed with a solid catalyst (e.g. an acid cation exchange resin). A mixture of NVR and a selected alcohol (such as 2-ethyl-1-hexanol) is heated in a furnace and injected into the fractionation tower at the point of the acid catalyst packing. Volatile reaction products (chiefly water and volatile esters) are taken off overhead as vapor and are condensed. The non-volatile ester product drops down the fractionating column. Appropriate boiling range fractions can be taken off the tower if required. The bottoms of the tower can be recycled back to the reaction zone to ensure completion of the reaction. Also, the tower bottoms can be fed to a vacuum distillation tower to be fractionated for appropriate viscosity range base oils.

One particularly important use for the esters of the invention is that of a base oil for a drilling mud. It is believed that the low relative cost and the low known potential for environmental impact of ester-based drilling muds makes this material highly desirable as an inhibitive drilling fluid. The inventors envision using NVR esters having a viscosity in the range of 1 - 20 cSt (at 25°C) as the base oil for the drilling mud, however, it is believed that an ester having a viscosity in the range of from 1 - 10 cSt (at 25°C) would serve most drilling functions adequately. The most preferred ester for a drilling mud base oil would have a viscosity in the range of from 1 - 4 cSt (at 25°C). The NVR ester is incorporated into drilling muds in the same proportions as conventional esters and oils are used. The mud formulation and the balance of the drilling mud composition would be conventional or largely conventional. For example, a highly suitable drilling mud can be prepared by adding 1 - 10 cSt (at 25°C) NVR ester (in lieu of a conventional ester base oil) to PETROFREE brand drilling mud ingredients which are commercially available from Baroid Drilling Fluids, Inc. of Houston, Texas.

The preferred NVR esters for use as drilling mud base oils are those esters prepared from the reaction of 2-ethyl-1-hexanol and/or neopentylglycol with the C₄ - C₆ monobasic carboxylic acids obtained from the processing vapors (overhead vapors) of NVR as obtained in the NVR batch processing techniques described above.

## Claims

1. A process for producing a complex ester compound suitable for use as a lubricant base oil, wherein the process comprises reacting one or more members of the group consisting of alcohols and polyols with the non-volatile residue by-product of the oxidation reaction of cyclohexane under conditions for promoting the formation of esters.

2. The process as claimed in Claim 1, wherein the non-volatile residue by-product is the material assigned Chemical Abstracts number CAS 6841-76-7 and is also known as NVR.

3. The process as claimed in Claim 1, wherein the alcohols and polyols are one or members selected from the group consisting of:
mono-alcohols of the general formula:
R¹OH
wherein R¹ is an alkyl group comprising from 1-25 carbons which can be straight, branched or cyclic in structure;
neopentylpolyols of the general formula: 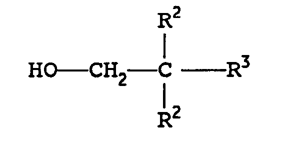 wherein R² represents one or more members selected from the group consisting of: hydrogen, a C₁ - C₆ alkyl which is straight chained or branched, and CH₂OH, and R³ represents a C₁ - C₃ alkyl group which is straight or branched, or CH₂OH;
neopentylpolyols of the general formula: 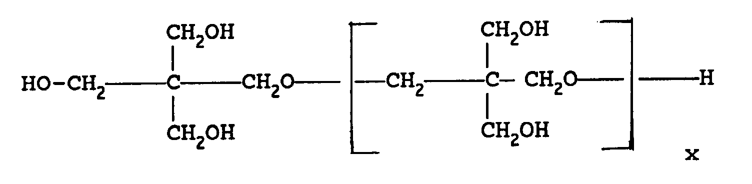 wherein **x** is an integer of from 0 to 4;
linear diols of the general formula:
HO-[R⁴O]_{z}H
wherein R⁴ represents a C₁-C₁₀ alkyl group which is straight or branched; and **z** is an integer of from 1 - 100;
polyglycols of the general formula: 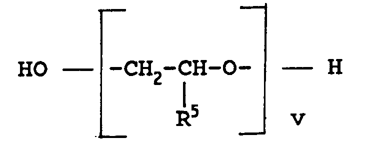 wherein R⁵ represents H, or a C₁-C₆ alkyl group which is straight or branched, and **v** represents an integer of from 1 - 50; and
polyglycerines of the general formula: 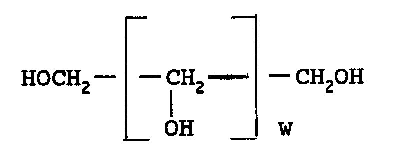 wherein **w** represents an integer of from 1 - 20.

4. The process as claimed in Claim 3, wherein said monoalcohols are one or more alcohols selected from the group consisting of methanol, ethanol, propanol, butanol, pentanol, hexanol, heptanol, octanol, nonanol, decanol, undecanol, and dodecanol, wherein said alcohols have an alkyl portion which may be straight, branched, or cyclic.

5. A process as claimed in Claim 3, wherein it is preferred that **z** is an integer of from 1 - 20 and that R⁴ represents a C₁ - C₆ alkyl group which is straight or branched.

6. A process as claimed in Claim 3, wherein said linear polyols are one or more members selected from the group consisting of ethyleneglycol, propyleneglycol, butyleneglycol, pentyleneglycol, hexyleneglycol, heptalyene glycol, nonaleneglycol, decaleneglycol, wherein the [R⁴O] moiety represented in the general formula of the linear diol repeats from 1 to 100 times.

7. A process as claimed in Claim 1, wherein said conditions for promoting ester formation comprise reacting the alcohol and/or diol and NVR in the presence of a catalyst for promoting the esterification reaction.

8. A process as claimed in Claim 7, wherein said catalyst is selected from the group consisting of strong acid catalysts, solid acid catalysts, and non-acid catalysts suitable for promoting the esterification reaction.

9. A process as claimed in Claim 8, wherein the preferred catalyst is the solid acid catalyst sulfonated polystyrene macroreticular acidic ion exchange resin.

10. A process as claimed in Claim 1, wherein said conditions for promoting ester formation comprise reacting the alcohols and/or polyols and non-volatile residue reactants at a temperature in the range of 0 - 250°C.

11. A process as claimed in Claim 10, wherein said temperature is in the range of 180 - 250°C.

12. A complex ester composition suitable for use as a lubricant base oil made by the process which comprises reacting one or more members of the group consisting of alcohols and polyols with the non-volatile residue by-product of the oxidation reaction of cyclohexane under conditions for promoting the formation of esters.

13. The composition as claimed in Claim 12, wherein the non-volatile residue by-product is the material assigned Chemical Abstracts number CAS 6841-76-7 and is also known as NVR.

14. The process as claimed in Claim 13, wherein said alcohols and polyols are one or members selected from the group consisting of:
mono-alcohols of the general formula:
R¹OH
wherein R¹ is an alkyl group comprising from 1-25 carbons which can be straight, branched or cyclic in structure;
neopentylpolyols of the general formula: 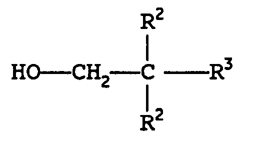 wherein R² represents one or more members selected from the group consisting of: hydrogen, a C₁ - C₆ alkyl which is straight chained or branched, and CH₂OH, and R³ represents a C₁ - C₃ alkyl group which is straight or branched, or CH₂OH;
neopentylpolyols of the general formula: 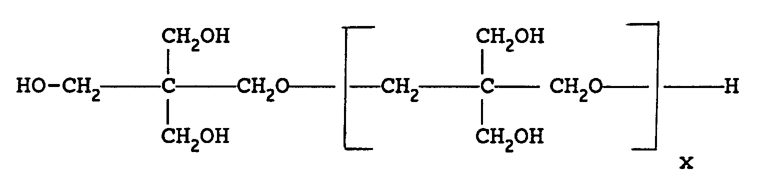 wherein **x** is an integer of from 0 to 4;
linear diols of the general formula:
HO-[R⁴O]_{z}H
wherein R⁴ represents a C₁-C₁₀ alkyl group which is straight or branched; and **z** is an integer of from 1 - 100;
polyglycols of the general formula: 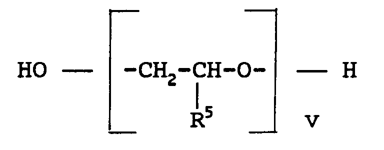 wherein R⁵ represents H, or a C₁-C₆ alkyl group which is straight or branched, and **v** represents an integer of from 1 - 50; and
polyglycerines of the general formula: 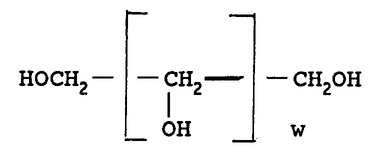 wherein **w** represents an integer of from 1 - 20.

15. The composition as claimed in Claim 14, wherein said monoalcohols are one or more alcohols selected from the group consisting of methanol, ethanol, propanol, butanol, pentanol, hexanol, heptanol, octanol, nonanol, decanol, undecanol, and dodecanol, wherein said alcohols have an alkyl portion which may be straight, branched or cyclic.

16. A composition as claimed in Claim 15, wherein it is preferred that **z** is an integer of from 1 - 20 and that R⁴ represents a C₁-C₆ alkyl group which is straight or branched.

17. A composition as claimed in Claim 14, wherein said linear polyols are one or more members selected from the group consisting of ethyleneglycol, propyleneglycol, butyleneglycol, pentyleneglycol, hexyleneglycol, heptalyene glycol, nonaleneglycol, decaleneglycol, wherein the [R⁴O] moiety represented in the general formula of the linear diol repeats from 1 to 100 times.

18. A composition as claimed in Claim 12, wherein said conditions for promoting ester formation comprise reacting the alcohols and/or polyols and non-volatile residue in the presence of a catalyst for promoting the esterification reaction.

19. A composition as claimed in Claim 18, wherein said catalyst is selected from the group consisting of strong acid catalysts, solid acid catalysts, and non-acid catalysts suitable for promoting the esterification reaction.

20. A composition as claimed in Claim 18, wherein the preferred catalyst is the solid acid catalyst sulfonated polystyrene macroreticular acidic ion exchange resin.

21. A composition as claimed in Claim 12, wherein said conditions for promoting ester formation comprise reacting the alcohols and/or polyols and non-volatile resin reactants at a temperature in the range of 0 - 250°C.

22. A process as claimed in Claim 20, wherein said temperature is in the range of 180 - 250°C.

23. A drilling mud, wherein the ester base oil for said drilling mud composition is a complex ester made by the process which comprises reacting under conditions for promoting ester formation an alcohol and/or polyol with the non-volatile residue by-product of the oxidation reaction of cyclohexane to produce cyclohexanol and cyclohexanone.

24. A drilling mud, as claimed in Claim 23, wherein said non-volatile residue by-product material is assigned Chemical Abstracts number CAS 6841-76-7 and is also known as NVR, and said ester has a viscosity in the range of from 1 - 20 cSt (at 25°C).

25. A drilling mud, as claimed in Claim 24, wherein the viscosity of said ester is in the range of from 1 - 10 cSt (at 25°C).

26. A drilling mud, as claimed in Claim 23, wherein said ester is obtained from the reaction of NVR and one or more members of the group consisting of 2-ethyl-1-hexanol and neopentyl glycol.

27. A drilling mud, as claimed in Claim 23, wherein said NVR comprises C₄ - C₆ monobasic carboxylic acids and said NVR is pretreated to concentrate the C₄ - c₆ monobasic carboxylic acids for reaction with said alcohols and/or polyols to form esters.

28. A drilling mud, as claimed in Claim 27, wherein said NVR is reacted with one or more members of the group consisting of 2-ethyl-1-hexanol and neopentyl glycol.

29. A complex ester composition comprising a mixture of esters and other reaction products obtained from the reaction of NVR and alcohols and/or polyols under conditions suitable for esterification, wherein the mixture of esters of the composition are represented by general formula: 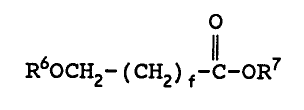 wherein R⁶ represents one or more of the moieties selected from the group consisting of:
-H ; 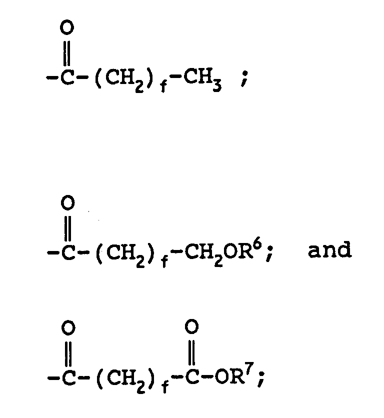
and wherein R⁷ represents one or more of the moieties selected from the group consisting of:
-CH₂-(CH₂)_{f}-CH₃; 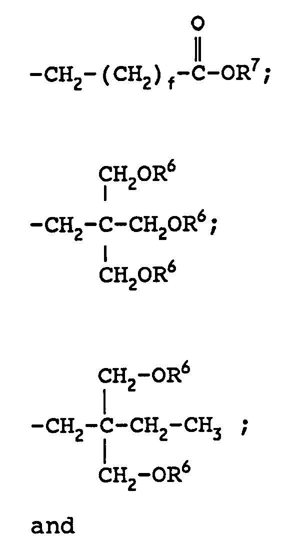 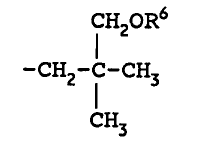
and **f** represents integers of from 2 through 4.
